# FASCICULE DE BREVET EUROPEEN

(11) **EP 1 164 947 B1**
(45) Date de publication et mention de la délivrance du brevet: **05.07.2006**
(21) Numéro de dépôt: 00912725.9
(22) Date de dépôt: 22.03.2000
(51) Int. Cl.: A61B 17/32

(54) **APPAREIL CHIRURGICAL A JET DE LIQUIDE SOUS PRESSION POUR LA REVASCULARISATION TRANSMYOCARDIALE**
CHIRURCHISCHE HOCHDRUCKFLÜSSIGKEITSSCHNEIDEVORRICHTUNG ZUR TRANSMYOKARDIALEN REVASKULARISATION
SURGICAL APPARATUS WITH PRESSURISED LIQUID JET FOR TRANSMYOCARDIAL REVASCULARIZATION

(30) Priorité: 22.03.1999 DE 19912844; 20.08.1999 FR 9910710
(43) Date de publication de la demande: 02.01.2002
(73) Titulaire: Eschmann Holdings Limited, Lancing, West Sussex BN15 8TJ (GB)
(72) Inventeur: GONON, Bertrand, F-69002 Lyon (FR)
(74) Mandataire: Metz, Paul
(86) Numéro de dépôt international: PCT/FR2000/000716
(87) Numéro de publication internationale: WO 2000/056233

(56) Documents cités:
- EP-A- 0 888 750
- WO-A-96/35469
- WO-A-98/55033
- WO-A-99/33510
- US-A- 5 735 815

## Description

La présente invention concerne un appareil chirurgical à jet de liquide sous pression comportant notamment une pièce à main. Cet appareil chirurgical sera particulièrement utilisé pour des interventions connues sous le nom de revascularisation transmyocardiale.

Le myocarde est un muscle du coeur qui en se contractant et en se relâchant successivement provoque le pompage du sang et son envoi dans la totalité du corps à travers le système vasculaire. Pour réaliser convenablement ces opérations fondamentales, le myocarde doit être convenablement oxygéné et alimenté en nutriments. Les apports du muscle sont assurés par un afflux de sang circulant dans le réseau des artères coronaires alimentant le myocarde.

Avec l'âge et une alimentation riche en graisse, une couche lipidique se dépose petit à petit contre les parois internes de ces artères coronaires. Ce dépôt finit par obstruer partiellement une ou plusieurs de ces artères, limitant l'irrigation d'une partie du myocarde. Lorsque la limitation est trop forte ou lors de l'obstruction, ce phénomène provoque l'infarctus souvent mortel ou tout du moins de très graves problèmes de santé et une dégradation importante de la qualité de vie. Les maladies cardio-vasculaires étant la plus grande cause de mortalité dans les pays industrialisés, la recherche médicale s'est fortement intéressée à ce problème.

En plus d'un traitement à base de médicaments, deux types d'interventions chirurgicales sont couramment pratiquées pour rétablir la circulation sanguine dans le myocarde : le pontage coronarien et l'angioplastie coronaire.

Le pontage coronarien consiste à prélever un morceau de veine par exemple dans la jambe du patient et à l'implanter entre l'aorte et un point de l'artère coronaire bouchée en aval de l'obstruction. On réalise ainsi une dérivation de l'artère bouchée, court-circuitant l'obstruction et rétablissant l'irrigation de la région ischémiée du myocarde en aval de l'obstruction. Pour pratiquer cette intervention chirurgicale, il est nécessaire d'arrêter le coeur du patient et de le remplacer temporairement par des machines. Pour cette raison entre autres, cette intervention présente de nombreux risques de complications.

L'angioplastie coronaire consiste à envoyer une sonde avec un petit ballon dans l'artère coronaire bouchée. Lorsque la sonde a traversé le bouchon lipidique, on gonfle le ballon afin d'écarter le dépôt de graisse et de le plaquer contre les parois de l'artère pour rétablir la circulation sanguine. Cette opération est souvent complétée par la pose d'extenseurs dits stents, implants métalliques permanents servant à maintenir ouvert un passage au sein de l'artère et à réduire les risques de rebouchage postérieur à l'intervention. Cette intervention est beaucoup moins traumatisante que le pontage et peut même parfois se dérouler sous anesthésie locale. Cependant, elle peut ne pas être réalisable si la plaque graisseuse est trop dure, si l'artère est trop petite ou si le dépôt est trop diffus. De plus, dans un nombre non négligeable de cas, l'artère se rebouche peu après l'intervention.

Les deux techniques précédentes, combinées avec la prise de médicaments et un régime alimentaire approprié, permettent de sauver de nombreux patients atteints de maladies cardio-vasculaires. Pourtant, pour 3 à 5% d'entre eux ces techniques sont insuffisantes. Les personnes concernées sont par exemple des patients ayant déjà subi un ou plusieurs pontages et/ou angioplasties qui se sont révélés inefficaces ou insuffisants. Il peut s'agir également de personnes présentant une ischémie trop sévère, diffuse ou inaccessible pour les interventions précédentes, ou bien possédant des obstructions dans des artères trop petites pour être pontées ou pour réaliser une angioplastie. Pour éviter à ces personnes l'ultime recours de la transplantation cardiaque, une nouvelle technique, la revascularisation transmyocardiale, a été récemment expérimentée.

Cette intervention consiste à percer plusieurs petits conduits à travers la paroi au droit de la zone ischémiée du myocarde entourant le ventricule gauche. Ces perforations provoquent une amélioration immédiate de l'irrigation de la zone ischémiée, mais elles ont surtout pour but de stimuler l'angiogenèse. En effet, après l'intervention, de nouveaux vaisseaux se développent autour des perforations remplaçant les artères obstruées.

Cette technique s'inspire du système d'oxygénation du coeur des reptiles qui ne possèdent pas de système d'artères coronaires. Chez ces animaux, le sang est directement amené du ventricule gauche au myocarde par l'intermédiaire de conduits endothéliaux. Les foetus utilisent un système identique avant le développement de leurs artères coronaires.

Les perforations de revascularisation transmyocardiale sont généralement pratiquées au moyen d'un faisceau laser généré par un appareil laser au dioxyde de carbone à haute puissance. Pour réaliser ce type d'interventions, de nombreux appareils laser ont été proposés dans l'état de la technique. On connaît par exemple les dispositifs laser et les procédés les utilisant, divulgués dans les demandes WO 98/49963, WO 98/38916, WO 98/31281, EP 0515867, EP 0876795, EP 0196519, EP 0856290, EP 0876796, EP 0858779, EP 0815798, EP 0836834, EP 0792624, EP 0797958, EP 0799604, EP 0801928.

On connaît également des dispositifs de perçage mécaniques, par exemple ceux décrits dans les demandes EP 0829239, EP 0792624, EP 0801928, EP 0807412, ou utilisant des énergies diverses telles que celles provenant de la haute fréquence (WO 98/38925, EP 0808607, WO 97/18768, WO 98/25533), des radiofréquences (WO 98/27877), ou des vibrations mécaniques (WO 98/16154).

L'intervention de revascularisation transmyocardiale se déroule généralement de la façon suivante. Le patient est d'abord placé sous anesthésie générale. Le chirurgien pratique une incision au niveau de son thorax afin d'exposer le myocarde au niveau du ventricule gauche. Contrairement à une intervention de pontage coronarien, le coeur du patient n'est pas arrêté et continue à battre pendant l'opération. Le chirurgien réalise ensuite des perforations à travers le myocarde jusqu'à l'intérieur du ventricule, à l'aide d'un laser piloté par ordinateur. On effectue entre 10 et 45 perforations espacées d'environ 1 cm et d'un diamètre d'environ 1 mm. Chaque perforation est réalisée avec une énergie moyenne comprise entre 30 et 60 Joules. Le nombre de perforations et l'énergie utilisée varient pour chaque patient en fonction de la taille du coeur et de la quantité de graisse autour du coeur.

La synchronisation entre l'application du rayon laser et le rythme cardiaque est très importante afin d'éviter une arythmie dangereuse pour la vie du patient. Le rayon doit être commandé entre deux battements de coeur lorsque le ventricule est rempli au maximum par le sang et est électriquement inactif (onde R de l'électrocardiogramme). Lorsque l'on forme les conduits dans le muscle pendant cette période, le sang présent joue le rôle d'une butée arrière, empêchant le laser d'endommager l'intérieur du ventricule et absorbant l'excès d'énergie.

Lorsque le ventricule se contracte, il se comporte comme une éponge, absorbant le sang à l'intérieur des perforations. Le sang peut ainsi s'infiltrer dans les tissus ischémiés.

A l'extérieur du coeur, l'extrémité des conduits se rebouche très rapidement en moins d'une minute si l'on arrête le saignement grâce à la pression du doigt du chirurgien ou de l'extrémité de l'appareil chirurgical. Les conduits doivent cependant rester ouverts à l'intérieur du muscle. Les tissus cicatriciels se forment en approximativement deux jours.

Bien que le flux sanguin dans le muscle soit immédiatement amélioré grâce aux conduits débouchant dans le ventricule, les résultats optimums ne sont obtenus qu'après la formation de petits vaisseaux à partir des perforations réalisant un nouveau réseau vasculaire irriguant le myocarde. Cette angiogenèse prend suivant les patients entre 3 mois et un an à partir de l'intervention.

Il est également possible de réaliser une revascularisation transmyocardiale par laser en perforant la paroi du myocarde de l'intérieur du ventricule. Il faut dans ce cas régler la puissance du laser pour que les perforations ne traversent pas complètement la paroi du muscle. L'appareil laser est amené jusqu'à sa position de travail à l'aide d'un cathéter enfilé le long de l'artère fémorale. L'intervention est beaucoup moins traumatisante, le coeur n'étant pas mis à jour.

Un appareil permettant de réaliser une intervention de ce type est par exemple décrit dans la demande WO 96/35469. Il s'agit d'un appareil de revascularisation transmyocardiale à fibre optique utilisant une source d'énergie laser, qui coulisse à l'intérieur de plusieurs cathéters concentriques de guidage et d'acheminement. Ce document évoque succinctement un remplacement possible de cette source d'énergie laser par un jet de fluide sous pression, ainsi que l'utilisation d'une source d'aspiration afin d'évacuer les débris hors du site opératoire.

La revascularisation transmyocardiale présente de nombreux avantages par rapport au pontage coronarien classique. Comme le coeur continue à battre pendant l'intervention, on évite de nombreuses complications pouvant être rencontrées après un pontage liées à l'arrêt et au redémarrage du coeur (hypotension, oedème, augmentation de poids, hémorragies...). De plus, le coût de l'intervention est significativement réduit par rapport à celui d'un pontage.

L'utilisation d'un faisceau laser comme outil de travail est la plus répandue. Cependant, elle n'est pas sans conséquence. En effet, la découpe par le rayon laser s'effectue en brûlant les tissus sur le trajet de l'impact. De ce fait, même si le rayon est extrêmement fin, la découpe n'est pas nette et est grandement traumatisante pour les tissus adjacents qui se nécrosent et meurent. Du fait de la nécrose des tissus autour des parois des conduits ainsi perforés, il a été observé que la plupart des conduits de revascularisation créés par laser avaient tendance à se reboucher à plus ou moins long terme, annulant les effets bénéfiques de l'intervention.

Il est de plus assez difficile de contrôler la puissance et l'effet du rayon pour qu'il ne cause pas de dégâts involontaires au myocarde. Une étude récente a rapporté que lors d'une intervention de revascularisation par laser, il peut se produire lors de la réalisation des perforations une destruction des nerfs du myocarde. Cette dénervation, même si elle diminue la douleur, peut se révéler très dangereuse pour le patient. En effet, il ne sent plus les douleurs servant d'alarme signalant une ischémie, ce qui l'expose à un infarctus fatal ou à une arythmie.

Le but de l'invention est de proposer un appareil permettant de pratiquer la revascularisation transmyocardiale n'utilisant pas de faisceau laser pour réaliser les perforations afin d'éviter tous les inconvénients précédemment cités.

A cette fin, le dispositif selon l'invention utilise un jet de liquide sous pression pour former à la manière d'une piqûre les canaux de revascularisation dans la paroi du myocarde. La revascularisation transmyocardiale peut se faire de la même façon qu'avec un laser. Elle présente tous les avantages précédemment évoqués. Elle peut être réalisée de l'extérieur du coeur sous la forme de conduits ouverts ou de l'intérieur du ventricule à l'aide d'un cathéter sous la forme de trous borgnes.

Mais, si la revascularisation par jet de liquide sous pression apporte tous les avantages de la revascularisation par laser, elle présente des avantages supplémentaires très importants.

Le travail de réalisation d'un conduit créé par l'extrémité d'une pièce à main chirurgicale ou un cathéter à liquide sous pression est parfaitement net et précis. Aucune brûlure ou nécrose n'affecte les parois du conduit et les tissus adjacents. De ce fait les perforations ont beaucoup moins tendance à se reboucher.

En plus de la précision de la perforation, la protection des tissus ne devant pas être détruits est beaucoup mieux assurée que dans le cas d'un laser. En effet, lorsque l'on forme un trou borgne, le liquide déjà présent dans la cavité crée un effet de matelas amortissant l'action du jet sous pression sur les tissus adjacents. Le risque de dénervation involontaire est de ce fait fortement réduit voire inexistant. Cet avantage est encore renforcé par l'effet de dissection que l'on peut obtenir avec l'appareil selon l'invention.

Le fait d'utiliser un liquide pour pratiquer les perforations présente un avantage supplémentaire apportant une très grande amélioration par rapport aux méthodes connues de revascularisation. A l'aide du dispositif selon l'invention, il devient possible d'injecter un liquide thérapeutique, directement au niveau des conduits dans le myocarde et simultanément à leur réalisation. Pour ce faire, il suffit d'utiliser un agent traitant comme liquide de travail du dispositif à jet de liquide sous pression selon l'invention. L'application du liquide traitant peut également être réalisée avant ou après la réalisation des perforations. L'agent traitant peut ainsi par exemple, mais non limitativement, être un agent préparant le muscle à la formation des conduits, favorisant le développement de nouveaux vaisseaux, limitant le rebouchage des perforations, évitant les complications post-opératoires, ou tout autre agent utile en cas d'intervention chirurgicale ou dans le traitement des maladies cardio-vasculaires.

Cette caractéristique de l'invention présente un avantage considérable car elle permet de combiner à la revascularisation transmyocardiale, les avantages d'un autre traitement expérimental par thérapie génique visant à développer la vascularisation des zones ischémiées du myocarde. Au cours de ce traitement, on injecte directement dans la région ischémiée du myocarde des millions de copies d'un gène favorisant la création de nouveaux vaisseaux appelé facteur de croissance vasculaire endothéliale (VEGF). Ce gène peut également être injecté non pas directement mais en utilisant un virus affaibli comme vecteur de transport.

Avec le dispositif selon la présente invention, un liquide contenant les copies de ce gène, le virus ou tout autre vecteur de celui-ci, peut être utilisé directement comme liquide de travail pour les perforations ou bien être appliqué au moyen de l'appareil selon l'invention tout de suite après le travail de réalisation des perforations. Cette application peut déclencher, accélérer ou accroître l'angiogenèse que l'on cherche à générer par l'intervention de revascularisation transmyocardiale.

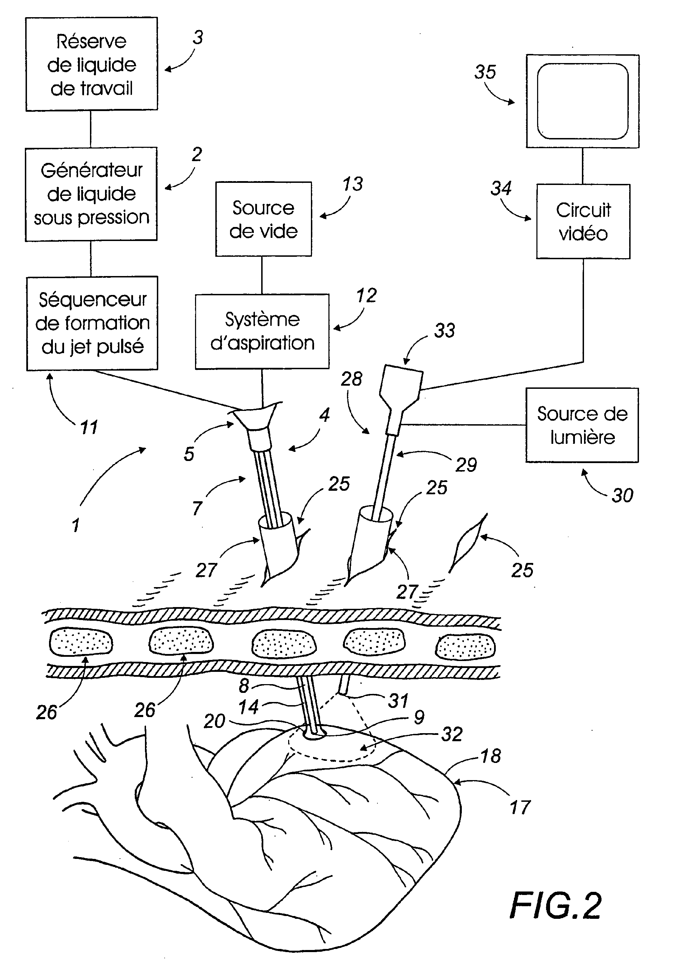

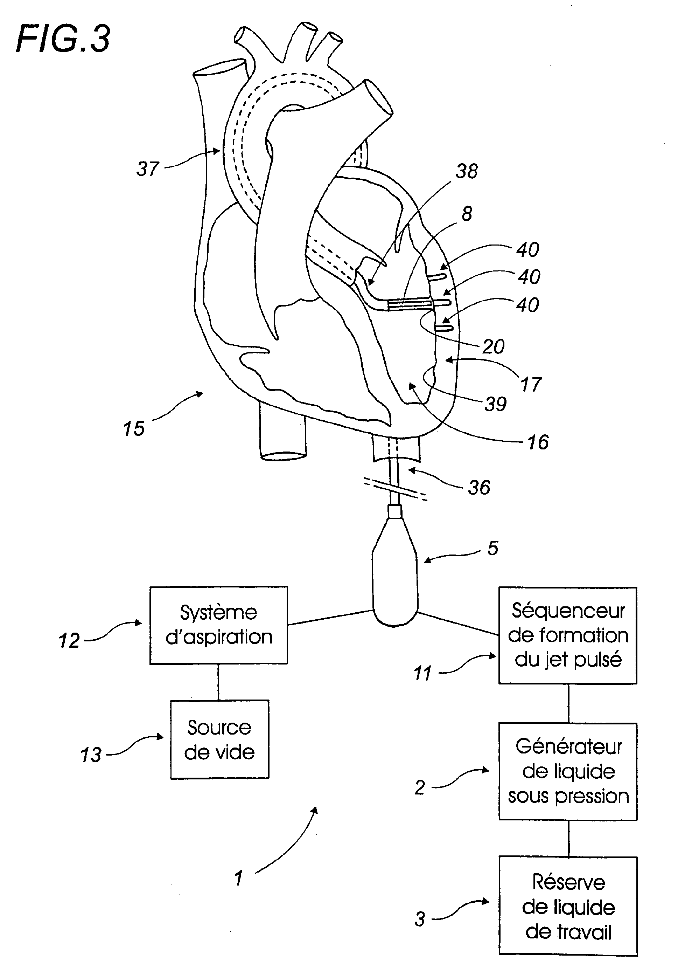

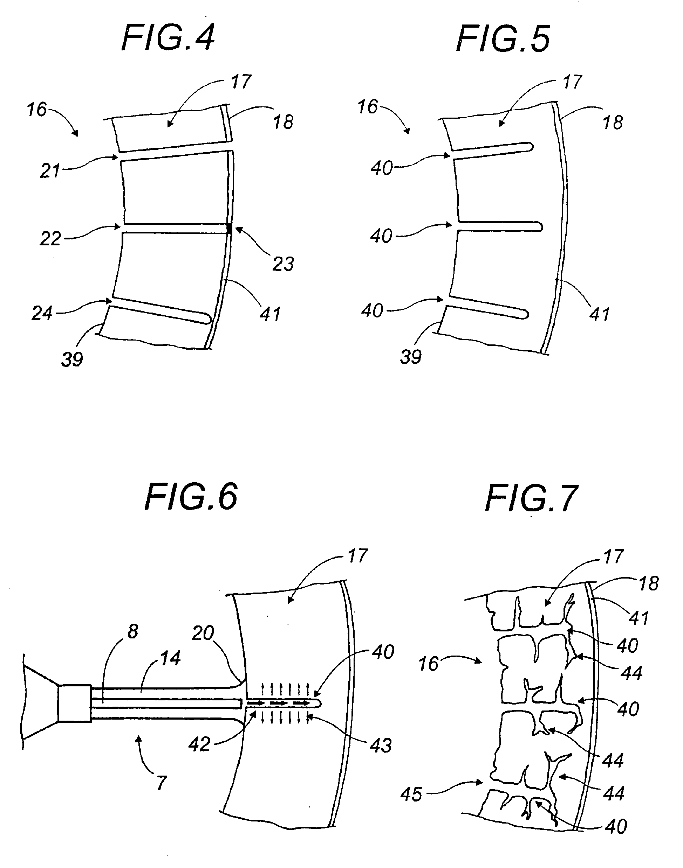

D'autres caractéristiques et avantages de l'invention apparaîtront à la lecture de la description détaillée qui va suivre de l'appareil chirurgical à jet de liquide sous pression pour la revascularisation transmyocardiale, description faite en référence aux dessins annexés, dans lesquels :

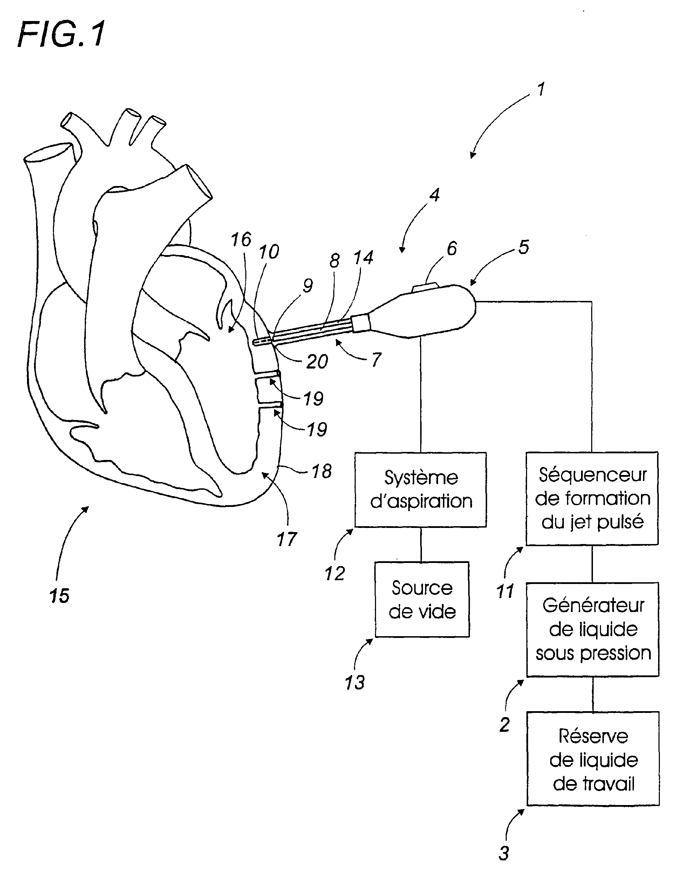

la figure 1 est un schéma explicatif simplifié d'un appareil chirurgical à liquide sous pression en cours d'intervention de revascularisation transmyocardiale selon l'invention, les perforations de la paroi du myocarde étant réalisées de l'extérieur du coeur par laparotomie ;
. la figure 2 est un schéma explicatif simplifié d'un appareil chirurgical à liquide sous pression réalisant une revascularisation transmyocardiale selon l'invention, les perforations de la paroi du myocarde étant réalisées de l'extérieur du coeur par laparoscopie ;
. la figure 3 est un schéma explicatif simplifié d'un appareil chirurgical à jet de liquide sous pression réalisant une revascularisation transmyocardiale percutanée selon l'invention, les perforations de la paroi du myocarde étant formées de l'intérieur du coeur au moyen d'un cathéter pénétrant dans le ventricule ;
. la figure 4 est une vue schématique en coupe d'une partie de la paroi du myocarde présentant des perforations de revascularisation transmyocardiale réalisées de l'extérieur du coeur au moyen de l'appareil selon l'invention ;
. la figure 5 est une vue schématique en coupe d'une partie de la paroi du myocarde présentant des perforations de revascularisation transmyocardiale réalisées de l'intérieur du coeur au moyen de l'appareil selon l'invention ;
. la figure 6 est un schéma explicatif de l'action du liquide de travail de l'appareil chirurgical selon l'invention au cours d'une intervention de revascularisation transmyocardiale ;
. la figure 7 est une vue en coupe schématique d'une partie de la paroi du myocarde où se produit une angiogenèse induite par une intervention de revascularisation transmyocardiale réalisée au moyen de l'appareil l'invention.

L'appareil chirurgical, selon l'invention, à jet de liquide sous haute pression pour la revascularisation transmyocardiale va maintenant être décrit de façon détaillée en référence aux figures 1 à 7. Les éléments équivalents, représentés sur les différentes figures, porteront les mêmes références numériques.

L'appareil chirurgical selon la présente invention a été schématiquement représenté sur les figures 1, 2 et 3. Il s'agit d'un appareil 1 capable d'envoyer un ou plusieurs jets de liquide sous pression contre la paroi du myocarde afin de créer des conduits de revascularisation.

Cet appareil comporte un générateur de liquide sous pression 2 relié à une réserve 3 de liquide de travail. Cette réserve peut être, par exemple, une poche en matière plastique contenant le liquide de travail enfermée dans une enceinte que l'on remplit par un gaz neutre afin de comprimer la poche et de mettre le liquide sous pression. La pression du jet de liquide généré peut être réglable afin de l'adapter aux besoins.

Le liquide de travail est amené à une pièce à main 4 permettant au chirurgien réalisant l'intervention de commander le déclenchement du jet de liquide de travail et de le diriger. La pièce à main 4 comporte un corps 5 ergonomique, permettant une préhension et un maniement aisés et pouvant présenter des organes de commande tels que par exemple un bouton-poussoir 6, ainsi qu'une extrémité active 7 par exemple celle d'un tube délivrant le jet liquide sous pression. Le tube d'extrémité 7, par exemple de forme cylindrique, comprend un conduit 8 à l'intérieur duquel passe le liquide de travail jusqu'à un orifice d'extrémité 9 par lequel jaillit le jet de liquide sous pression 10 afin de réaliser un travail chirurgical de découpe et plus particulièrement ici de pénétration dans le muscle cardiaque à la manière d'une piqûre par une aiguille.

Pour une meilleure efficacité, l'appareil chirurgical selon la présente invention est de préférence un appareil à jet pulsé envoyant le liquide sous pression par un tir sous la forme d'un train discontinu d'impulsions constituant des jets élémentaires de liquide sous pression. Un tir peut être constitué d'une seule impulsion. Dans ce mode de réalisation, l'appareil chirurgical comporte en outre un séquenceur 11 permettant la formation du jet pulsé et commandant ses paramètres.

L'appareil chirurgical 1 comporte de préférence un système d'aspiration 12 relié à une source de vide 13, par exemple le circuit général de vide de l'hôpital. Dans le mode de réalisation représenté sur la figure 1, le système d'aspiration se termine par un conduit d'aspiration 14, par exemple de forme générale cylindrique, situé au niveau de l'extrémité 7 et concentrique au conduit 8. Le conduit d'aspiration 14, débouchant à proximité de la zone d'intervention permet d'aspirer le liquide de travail et les petits débris au cours du travail de perforation des conduits de revascularisation, améliorant ainsi l'efficacité de la perforation et la visibilité de la zone d'intervention pour le chirurgien.

Selon une variante préférentielle, le conduit de passage du liquide sous pression est mis à certains moments, en fonction des caractéristiques du jet pulsé, en communication avec le système d'aspiration afin d'améliorer la forme du train des impulsions de liquide sous pression et selon une originalité du procédé breveté par ailleurs.

L'appareil chirurgical selon la présente invention peut être utilisé pour réaliser les trois types connus d'intervention de revascularisation transmyocardiale : par laparotomie, par laparoscopie ou bien par voie percutanée. Trois variantes du dispositif selon l'invention adaptées à chacune de ces interventions ont été représentées respectivement sur les figures 1, 2 et 3.

Pour réaliser l'intervention de revascularisation par laparotomie au moyen de l'appareil selon l'invention, le chirurgien commence par pratiquer une incision dans le thorax du patient, sous anesthésie générale, et à exposer son coeur 15 au niveau du ventricule gauche 16 afin de pouvoir accéder à la zone ischémiée du myocarde 17. Généralement, cette zone se situe dans la partie basse du myocarde entourant le ventricule gauche 16.

Après avoir repéré la zone à revasculariser, le chirurgien positionne la pièce à main de façon que l'extrémité active 7 soit en contact avec la paroi extérieure 18 du myocarde 17. Il déclenche alors le tir de liquide sous pression afin de créer un premier conduit 19 de revascularisation. Le liquide sous pression percute les tissus et pénètre dans ceux-ci pour y former un conduit.

Lorsqu'il pratique une intervention de revascularisation en partant de l'extérieur du coeur, le chirurgien doit réaliser des conduits 19 traversant complètement le myocarde 17 et débouchant dans le ventricule gauche 16, afin de mettre en communication les tissus ischémies du myocarde avec le sang oxygéné présent à l'intérieur du ventricule.

Les perforations peuvent être réalisées par un seul tir mono-impulsion de liquide sous pression ou bien par plusieurs impulsions successives d'un même tir. Il suffit de modifier les réglages des paramètres de tir de l'appareil à jet de liquide sous pression.

L'appareil selon l'invention comporte un moyen de positionnement et d'immobilisation de la pièce à main pour la maintenir pendant le travail de perforation. Il est en effet important que l'extrémité active 7 ne bouge pas entre deux impulsions successives de liquide. Ce maintien peut être réalisé par un moyen d'immobilisation quelconque. Il peut s'agir d'un moyen mécanique d'ancrage de l'extrémité active 7 contre la paroi 18 du myocarde, par exemple à l'aide de griffes, et/ou d'un moyen mécanique adapté d'immobilisation du corps de la pièce à main.

Le moyen d'immobilisation et d'ancrage peut également utiliser le système d'aspiration de l'appareil chirurgical. On peut en effet imaginer un dispositif à pattes d'ancrage reliées au circuit de vide ou bien une jupe 20, légèrement évasée, prolongeant le conduit extérieur d'aspiration 14 de l'extrémité 7. Lorsque l'extrémité active 7 est en position, au contact ou proche de la paroi du myocarde 17, le chirurgien déclenche l'aspiration qui plaque l'extrémité du tube contre la paroi 18, réalisant ainsi l'immobilisation nécessaire à la précision de la perforation. D'autres variantes peuvent être imaginées sans sortir du cadre de l'invention.

Lorsque le chirurgien pratique une intervention de revascularisation transmyocardiale de l'extérieur du coeur, il peut utiliser le système d'aspiration de l'appareil chirurgical. Le liquide de travail, après avoir percuté le myocarde, est aspiré à l'intérieur de l'appareil chirurgical au moyen du conduit d'aspiration 14. L'aspiration augmente l'efficacité de l'appareil pour réaliser les perforations. En effet, si le liquide de travail s'accumule dans un conduit en cours de formation, le ou les tir(s) suivant(s) ne vont pas percuter les tissus du myocarde situés au fond du conduit, mais une couche épaisse de liquide qui amortit le choc et diminue la pression du jet et de ce fait l'efficacité du travail perforant. Le conduit est tout de même réalisé, mais la vitesse diminue.

L'utilisation de l'aspiration présente un autre avantage très pratique pour le chirurgien : il est, grâce à l'aspiration, immédiatement averti de la fin du travail de perforation d'un conduit de revascularisation 19. En effet, lorsque le conduit 19 est terminé et débouche dans le ventricule 16, le sang présent dans le ventricule pénètre dans celui-ci et est aspiré par le conduit d'aspiration 14. Le liquide aspiré prend alors une coloration rouge sang avertissant le chirurgien. Contrairement à la revascularisation par laser, il n'est pas nécessaire d'utiliser un autre instrument afin de vérifier que le conduit débouche dans le ventricule.

Lorsque le premier conduit 19 de revascularisation est terminé, le chirurgien retire l'appareil chirurgical et rebouche l'extrémité extérieure de ce conduit pour éviter une perte excessive de sang. A cet effet, il obture le passage par une pression de son doigt ou de la tête de l'appareil chirurgical sur le bord extérieur du conduit provoquant la coagulation au niveau de l'orifice. Il peut évidemment utiliser un autre instrument pour provoquer cette coagulation qui se réalise très rapidement.

Le chirurgien déplace ensuite l'appareil à jet de liquide pour le positionner en face de la position de réalisation du conduit de revascularisation suivant et réalise la perforation de la même façon que précédemment selon un motif prédéfini. Il effectue ainsi sur la zone ischémiée du muscle entre 5 et 30 perforations espacées par exemple d'environ 1 cm. Le nombre de perforations varie pour chaque patient en fonction de la taille du coeur, de l'étendue de la zone ischémiée et de la quantité de graisse autour du coeur.

Avantageusement, le nombre de conduits de revascularisation à effectuer est moins important que lorsque l'intervention est réalisée à l'aide d'un laser, les conduits ne se nécrosant pas et ayant de ce fait beaucoup moins tendance à se reboucher par la suite.

Lorsque le ventricule se contracte, il se comporte comme une éponge, forçant le sang à pénétrer à l'intérieur des perforations et à s'infiltrer dans les tissus ischémiés.

La figure 4 illustre trois conduits de revascularisation créés à partir de la paroi extérieure 18 du myocarde 17 au moyen de l'appareil selon l'invention. Ces trois conduits sont à trois stades différents de leur évolution. Le conduit 21 vient juste d'être percé. Il traverse complètement le myocarde et est débouchant à la fois sur sa face intérieure et sa face extérieure.

Pour le conduit 22, le phénomène de coagulation vient de débuter du côté extérieur 18 du myocarde, sous l'effet d'une pression extérieure d'obturation. Un caillot 23 de sang coagulé bouche l'orifice extérieur du conduit 22.

Pour le conduit 24, le phénomène de coagulation est terminé. Les tissus se sont reformés, rebouchant définitivement l'extrémité extérieure du conduit.

La synchronisation entre les tirs de jets de liquide sous pression pour la formation des canaux et le rythme cardiaque semble être moins importante que dans le cadre d'une intervention par laser. Cependant, pour des raisons de prudence, il est raisonnable de continuer à synchroniser le jet avec le rythme cardiaque afin de tirer entre deux battements de coeur lorsque le ventricule est rempli au maximum par le sang et est électriquement inactif. On limite ainsi au maximum les risques de fibrillation ou autres arythmies. Néanmoins, on peut envisager d'utiliser l'appareil chirurgical selon l'invention pour réaliser une telle intervention de revascularisation sans synchronisation des tirs de liquide sous pression avec le rythme cardiaque.

L'appareil chirurgical 1 selon la présente invention peut donc comporter en outre un module (non représenté) de synchronisation des tirs de jets de liquide sous pression avec le rythme cardiaque du patient.

L'intervention de revascularisation transmyocardiale peut également être réalisé par laparoscopie au moyen de l'appareil selon l'invention comme représenté sur la figure 2. La revascularisation se fait toujours en partant de l'extérieur du coeur. Cependant l'intervention est beaucoup moins traumatisante pour le patient, car son coeur n'est pas mis à nu et seules deux ou trois petites incisions sont pratiquées par le chirurgien.

La première étape de l'intervention consiste pour le chirurgien à pratiquer sur le thorax du patient des incisions 25 de petite taille, situées entre les côtes 26 à un endroit permettant d'atteindre la zone ischémiée du myocarde. Le chirurgien insère ensuite dans ces incisions des trocarts intermédiaires 27 afin de maintenir écartés les bords des incisions et de servir de guide pour les instruments. On peut utiliser pour réaliser cette intervention des trocarts 27 de diamètre standard, c'est-à-dire par exemple de 5 mm ou de 10 mm.

Les incisions sont au moins au nombre de deux afin de permettre la pénétration jusqu'au coeur de l'extrémité active de l'instrument chirurgical selon l'invention et d'un moyen 28 de vision, de repérage et d'éclairage pour piloter l'intervention sans qu'il soit nécessaire d'ouvrir le thorax du patient. Ces incisions peuvent être plus nombreuses, préférentiellement au nombre de trois, afin d'augmenter les angles et les points d'accès à la zone ischémiée du myocarde. Afin de limiter le nombre d'incisions nécessaires, l'extrémité active de l'instrument chirurgical et le moyen de repérage peuvent être permutés au cours de l'intervention.

Les trocarts 27 permettent également le passage et le guidage de nombreux autres instruments chirurgicaux et médicaux nécessaires à l'intervention, que ce soit avant ou après la formation des canaux de revascularisation.

L'appareil chirurgical selon l'invention permettant de réaliser cette intervention est un appareil 1 à jet de liquide sous pression similaire à celui précédemment décrit. Seule l'extrémité 7 change. En effet, celle-ci doit être adaptée pour passer à l'intérieur d'un trocart 27 et pour venir au contact de la paroi extérieure 18 du myocarde. Le tube d'extrémité 7 doit donc être de longueur plus importante. Il peut de plus, être articulé et orientable afin de pouvoir se positionner à l'endroit approprié du myocarde selon une inclinaison appropriée, ce système étant commandé de l'extérieur.

L'extrémité active 7 comporte également un système d'ancrage pour réaliser son immobilisation lors de la perforation d'un canal de revascularisation. Il peut s'agir par exemple comme précédemment d'un moyen mécanique quelconque d'ancrage, ou d'un moyen utilisant le système d'aspiration de l'instrument tel que par exemple la jupe 20.

Le moyen 28 de vision, de repérage et d'éclairage est par exemple constitué d'un thoracoscope présentant une longue tige d'extrémité 29 pouvant être glissée dans l'un des trocarts 27. Le thoracoscope comprend un système d'éclairage qui transmet, au moyen d'un faisceau de fibres optiques longeant la tige 29, la lumière d'une source lumineuse 30 jusqu'à l'extrémité 31 de la tige 29 et éclaire ainsi une zone 32 du myocarde à perforer. Il comprend également une caméra 33 qui grâce à un système vidéo-optique disposé dans la tige 29 forme des images correspondant à la zone éclairée 32 et les transmet par l'intermédiaire d'un circuit vidéo 34 à un écran 35.

Le chirurgien suit et dirige l'intervention en visualisant la région à perforer du myocarde et l'extrémité des instruments sur l'écran 35, l'intervention pouvant être synchronisée avec le rythme cardiaque et/ou assistée par ordinateur.

L'intervention se déroule de la façon suivante. Le chirurgien commence par repérer sur l'écran et à baliser la zone à revasculariser. Il l'éclaire au moyen du thoracoscope. Il positionne ensuite l'extrémité de l'appareil chirurgical dans cette zone, près de la paroi externe 18 du myocarde. Il immobilise l'extrémité active de l'appareil chirurgical au moyen du système d'ancrage et réalise le premier conduit de revascularisation à l'aide d'un ou de plusieurs tirs de liquide sous pression. Lorsque le conduit débouche à l'intérieur du ventricule, le chirurgien stoppe l'hémorragie en provoquant la coagulation de l'extrémité extérieure du conduit de revascularisation, en exerçant une pression avec la tête de l'instrument chirurgical. Il peut également utiliser un autre instrument à cet effet, par exemple un matériau absorbant retenu par une pince insérée à la place de l'appareil chirurgical dans le trocart 27. Les perforations suivantes sont réalisées de la même façon après avoir déplacé de façon appropriée l'extrémité active de l'appareil chirurgical.

Les conduits de revascularisation obtenus par laparoscopie sont semblables à ceux obtenus par laparotomie et sont représentés sur la figure 4.

Le dernier type d'intervention de revascularisation transmyocardiale est représenté sur la figure 3. L'intervention est cette fois réalisée, au moyen de l'appareil selon l'invention, en partant de l'intérieur du ventricule et se déroule de la façon suivante.

Le chirurgien introduit un cathéter de guidage 36 dans l'artère fémorale. Une autre artère, comme par exemple l'artère du bras, peut aussi être utilisée. Il fait progresser le cathéter 36 jusqu'à l'extrémité inférieure de la crosse de l'aorte 37. L'extrémité 7 de la pièce à main 4 est cette fois constituée d'un flexible 38 comprenant le conduit 8 transportant le liquide de travail. Le flexible 38 est introduit et guidé dans le cathéter jusqu'à la base de l'aorte, puis est poussé délicatement à travers le système valvulaire aortique jusqu'à ce que son extrémité pénètre dans le ventricule.

Le flexible comporte à son extrémité libre un dispositif de repérage (non représenté) comprenant un capteur permettant de voir en trois dimensions la zone à revasculariser avant le début des tirs. Le repérage de la zone de tir ainsi que la surveillance de l'intervention par le chirurgien peuvent être réalisés par un autre moyen sans sortir du cadre de l'invention.

Le chirurgien approche l'extrémité du flexible de la paroi interne 39 du coeur et plus particulièrement du ventricule gauche 16, à l'endroit où la première perforation doit être réalisée. L'extrémité active du flexible est alors plaquée contre la paroi 39 et maintenue en position par un dispositif d'ancrage par exemple semblable à ceux précédemment décrits. Le chirurgien effectue ensuite la première perforation 40. Puis il déplace l'extrémité active du flexible et réalise les perforations suivantes de la même façon.

Lorsque la revascularisation est réalisée à partir de l'intérieur du coeur, l'appareil à jet de liquide selon l'invention doit être réglé de manière à former des conduits borgnes 40, c'est-à-dire ne traversant pas toute l'épaisseur du myocarde. L'épaisseur de la paroi du myocarde étant comprise en moyenne entre 10 et 20 mm, le chirurgien réalise des conduits ayant de préférence entre 3 et 5 mm de longueur. Avec cette marge de sécurité, il est sûr de ne pas transpercer la paroi du muscle tout en assurant une bonne revascularisation. Le conduit, formé de l'intérieur du ventricule, alimente directement les tissus ischémiés avec le sang oxygéné présent dans le ventricule gauche 16. L'épicarde 41 est laissé intact, ce qui évite les problèmes d'hémorragie pouvant être dangereux.

Un exemple de conduits de revascularisation 40, réalisés de l'intérieur du coeur au moyen de l'appareil selon l'invention, a été représenté sur la figure 5. On peut ainsi les comparer avec les conduits de la figure 4, formés à partir de la paroi extérieure du myocarde par laparotomie ou laparoscopie.

Avec l'appareil chirurgical à jet de liquide sous pression selon l'invention, on peut avantageusement garantir une profondeur de perforation constante déterminée par les paramètres choisis par le chirurgien. Le diamètre de l'orifice 9 de sortie du jet de liquide, la pression du liquide de travail et la durée de l'impulsion de tir sont les trois paramètres influant sur la profondeur des perforations.

Le diamètre de l'orifice de sortie est choisi par exemple entre 0,1 et 0,5 mm. Il est de préférence égal à 0,3 mm.

La pression du liquide de travail est préférentiellement comprise entre 10 et 50 bar. Elle est de préférence égale à 20 bar.

La durée de l'impulsion de tir est de préférence comprise entre 100 et 1000 ms. Elle est de préférence égale à 200 ms.

Afin de ne pas provoquer d'arythmie dangereuse, la pression du liquide de travail ne doit pas être trop importante. Il est préférable d'utiliser un orifice 9 de diamètre relativement grand et une pression plus faible plutôt que l'inverse.

Lorsque le chirurgien a choisi et réglé la valeur de ces trois paramètres, il est certain d'obtenir des perforations de diamètre et de profondeur quasiment identiques pendant toute la durée de l'intervention. Les perforations restent également semblables d'un patient à l'autre.

L'appareil chirurgical à jet de liquide sous pression présente un autre avantage très intéressant pour le chirurgien et ne pouvant pas être obtenu avec un laser. En modifiant les trois paramètres de fonctionnement de l'appareil selon l'invention, le chirurgien peut réaliser un travail de dissection plus ou moins sélectif. Il peut, par exemple, diminuer la pression du liquide de travail pour que seuls les tissus les plus mous soient traversés par le jet. Comme ils sont plus durs que la paroi du myocarde, les artères ou les nerfs traversant le conduit de revascularisation peuvent ainsi être laissés intacts. Le chirurgien peut donc obtenir très simplement soit une perforation par pénétration parfaitement nette, soit un effet de dissection plus ou moins sélective selon les valeurs choisies pour les paramètres.

Le liquide de travail utilisé est de préférence du sérum physiologique. De ce fait la découpe est complètement stérile et non agressive pour les tissus. On limite ainsi les problèmes de stérilisation des aiguilles et appareils classiques de revascularisation et on réduit les risques de complications.

D'autres liquides peuvent évidemment être utilisés en tant que fluide de travail comme par exemple une solution saline, une solution de glucose, de Ringer-lactate, d'hydroxy-éthyl-amidon ou un mélange de ces solutions.

On peut également employer un liquide possédant une activité thérapeutique qui se trouve directement injecté dans chaque perforation dès sa formation. L'action du liquide ainsi appliqué est bien plus efficace que lorsqu'il est simplement amené par le sang.

On peut imaginer par exemple l'utilisation d'un agent préparant le muscle à la formation des canaux, favorisant le développement de nouveaux vaisseaux, limitant le rebouchage des perforations, évitant les complications post-opératoires, ou de tout autre agent utile. Un mélange, une solution, une dispersion, une suspension ou une émulsion de ceux-ci dans un liquide de base peut également être employé tant que sa viscosité reste compatible avec son utilisation en tant que fluide de travail pour l'appareil chirurgical.

Cette caractéristique de la présente invention permet avantageusement d'injecter dans les perforations un facteur de croissance vasculaire endothéliale (VEGF), seul ou avec un vecteur quelconque, afin de combiner à la revascularisation transmyocardiale un traitement par thérapie génique visant à développer l'angiogenèse.

On peut aussi envisager d'utiliser l'appareil à jet de liquide selon l'invention pour appliquer un agent thérapeutique avant ou après la formation des canaux de revascularisation.

La figure 6 illustre de façon schématique le mode d'action du fluide de travail. La plus grande partie du fluide, symbolisée par les flèches épaisses 42, est projetée sous pression dans la direction du canal 40 de revascularisation. Elle percute ainsi les tissus du myocarde 17 pour y créer le conduit 40.

Une petite partie du fluide, représentée par les flèches minces 43, diffuse à travers les parois transversales du conduit 40 et pénètre dans les tissus adjacents du myocarde qui se comportent comme une éponge. Ces tissus ne subissent pas de dommages car la pression du fluide est très faible perpendiculairement à la direction longitudinale du conduit. Le liquide de travail pénètre ainsi dans toutes les veinules et artérioles traversant le conduit de revascularisation, les débouche et éventuellement y dépose l'agent thérapeutique qu'il contient.

On a représenté sur la figure 7 le phénomène d'angiogenèse se produisant après une intervention de revascularisation transmyocardiale au moyen de l'appareil chirurgical selon l'invention. De minuscules vaisseaux 44 se forment à partir des perforations 40 et réalisent un nouveau réseau vasculaire irriguant le myocarde avec le sang 45 contenu dans le ventricule 16.

Si les trois types d'intervention de revascularisation sont réalisables avec l'appareil à jet de liquide sous pression selon l'invention, ils ne présentent pas le même degré de risques per- et post-opératoires. Le niveau de risque, ainsi que le coût de l'intervention diminuent de la laparotomie, à la laparoscopie, puis à la revascularisation par voie percutanée. Ces interventions sont en effet de moins en moins traumatisantes. Elles durent de moins en moins longtemps et le patient peut sortir plus rapidement de l'hôpital. L'intervention de revascularisation transmyocardiale, au moyen de l'appareil selon l'invention, par voie percutanée est donc préférentielle.

## Revendications

1. Appareil chirurgical à jet de liquide sous pression permettant de réaliser une intervention de revascularisation transmyocardiale au cours de laquelle on creuse des canaux de revascularisation du muscle cardiaque dans le muscle cardiaque, appareil présentant un générateur (2) de liquide sous pression, une réserve (3) de liquide de travail et une pièce à main (4), le générateur (2) étant relié à la réserve de liquide et à la pièce à main, **caractérisé en ce que** la pièce à main (4) présente une extrémité active (7) comprenant un conduit (8) et un orifice (9) de passage du liquide de travail relié au générateur (2) à travers un séquenceur (11) et une entrée d'aspiration reliée à travers un système d'aspiration (12) à une source de vide (13), **en ce qu'**il délivre un tir sous la forme d'une ou plusieurs impulsions de liquide de travail sous pression et **en ce que** le liquide de travail contient un agent thérapeutique.

2. Appareil selon la revendication 1 **caractérisé en ce que** le liquide de travail contient du sérum physiologique.

3. Appareil selon la revendication 1 **caractérisé en ce que** le liquide de travail contient une solution saline ou une solution de glucose ou une solution de Ringer-lactate ou une solution d'hydroxyéthyl-amidon ou un mélange de ces solutions.

4. Appareil selon la revendication 1 **caractérisé en ce que** l'agent thérapeutique est un agent préparant le muscle cardiaque à la formation de canaux de revascularisation.

5. Appareil selon la revendication 1 **caractérisé en ce que** l'agent thérapeutique est un agent favorisant le développement de nouveaux vaisseaux.

6. Appareil selon la revendication 1 **caractérisé en ce que** l'agent thérapeutique est un facteur de croissance vasculaire endothéliale.

7. Appareil selon la revendication 1 **caractérisé en ce que** l'agent thérapeutique est un agent de traitement par thérapie génique.

8. Appareil selon la revendication 1 **caractérisé en ce qu'**il comporte un moyen de synchronisation des impulsions avec le rythme cardiaque.

9. Appareil selon la revendication 1 **caractérisé en ce que** la réserve (3) de liquide est contenue dans une poche souple enfermée dans une enceinte sous pression.

10. Appareil selon la revendication 1 **caractérisé en ce que** la partie active (7) comporte un dispositif d'ancrage dans le muscle cardiaque en vue de son immobilisation à l'endroit où un conduit de revascularisation doit être réalisé.

11. Appareil selon la revendication 1 **caractérisé en ce que** la partie active (7) est entourée d'une jupe (20) reliée au système d'aspiration (12), pour son maintien par dépression contre la paroi du coeur à l'endroit où un conduit de revascularisation doit être réalisé.

12. Appareil chirurgical selon la revendication 1 **caractérisé en ce que** l'extrémité active (7) est celle d'un flexible (38) coulissant dans un cathéter de guidage (36) jusqu'à l'intérieur du coeur où un conduit de revascularisation doit être réalisé.

13. Appareil chirurgical selon la revendication 1 **caractérisé en ce que** l'extrémité active (7) est susceptible d'être introduite dans un trocart (27) traversant la poitrine jusqu'à la zone d'intervention.

14. Appareil chirurgical selon la revendication 1 **caractérisé en ce que** le conduit (8) de passage du liquide de travail peut être mis momentanément à la fin d'une impulsion en communication avec le système d'aspiration (12).

15. Appareil chirurgical à jet de liquide sous pression selon la revendication 1 **caractérisé en ce que** la pression du liquide de travail est comprise entre 10 et 50 bar.

16. Appareil chirurgical selon la revendication 1 **caractérisé en ce que** le diamètre de l'orifice (9) de sortie du liquide de travail est compris entre 0,1 et 0,5 mm.

17. Appareil chirurgical selon la revendication 1 **caractérisé en ce que** la durée de l'impulsion d'un tir à une seule impulsion de liquide de travail est comprise entre 100 et 1000 ms.

## Claims

1. Pressurised liquid jet surgical apparatus for transmyocardial revascularization operations, involving the creation of revascularization channels in the cardiac muscle, the apparatus comprising a generator (2) of pressurised liquid, a tank (3) of working liquid and a handpiece (4), the generator (2) being connected to the store of liquid and the handpiece, **characterised in that** the handpiece (4) has an active extremity (7) comprising a pipe (8) and an opening (9) for the working liquid connected to the generator (2) via a sequencer (11) and a suction inlet connected by a suction system (12) to a vacuum source (13), and which produces a jet in the form of one or more pulses of pressurised working liquid containing a therapeutic agent.

2. Apparatus as described in claim 1 **characterized by** the fact that the working liquid contains physiological serum.

3. Apparatus as described in claim 1 **characterized by** the fact that the working liquid contains a salt solution or a glucose solution or a Ringer-lactate solution or a hydroxy-ethyl-starch solution or a mixture of these solutions.

4. Apparatus as described in claim 1 **characterized by** the fact that the therapeutic agent is an agent predisposing the cardiac muscle to the formation of revascularization channels.

5. Apparatus as described in claim 1 **characterized by** the fact that the therapeutic agent is an agent which promotes the development of new vessels.

6. Apparatus as described in claim 1 **characterized by** the fact that the therapeutic agent is a vascular endothelial growth promoter.

7. Apparatus as described in claim 1 **characterized by** the fact that the therapeutic agent is a gene therapy agent.

8. Apparatus as described in claim 1 **characterized by** the fact that it comprises a means of synchronization of pulses with the heartbeat.

9. Apparatus as described in claim 1 **characterized by** the fact that the store (3) of liquid is contained in a flexible bag enclosed in a pressurised casing.

10. Apparatus as described in to claim 1 **characterized by** the fact that the active extremity (7) comprises a device for anchoring it to the cardiac muscle at the place where a revascularization conduit is to be made.

11. Apparatus as described in claim 1 **characterized by** the fact that the active extremity (7) is surrounded by a skirt (20) connected to the suction system (12), which enables it to be held in place by negative pressure against the wall of the heart at the place where a revascularisation conduit is to be made.

12. Surgical apparatus as described in claim 1 **characterized by** the fact that the active extremity (7) is a hose (38)which slides into a guide catheter (36)and then into the heart in which a revascularisation conduit is to be made.

13. Surgical apparatus as described in claim 1 **characterized by** the fact that the active extremity (7) may be introduced into a trocar (27) passing through the chest up to the zone of intervention.

14. Surgical apparatus as described in claim 1 **characterized by** the fact that the conduit (8) through which the working liquid flows can be temporarily placed in communication with the suction system at the end of a pulse (12).

15. Pressurised liquid jet surgical apparatus as described in claim I **characterized by** the fact that the pressure of the working liquid is between 10 and 50 bar.

16. Surgical apparatus as described in claim 1 **characterized by** the fact that the diameter of the opening (9) of the outlet of the working liquid is between 0,1 and 0,5 mm.

17. Surgical apparatus as described in claim 1 **characterized by** the fact that the duration of the pulse of a single jet of working liquid is between 100 and 1000 ms.

## Patentansprüche

1. Chirurgisches Hochdruck-Flüssigkeitsstrahlgerät, das die Durchführung einer transmyokardialen Revaskularisation ermöglicht, in deren Verlauf man Kanäle zur Revaskularisierung des Herzmuskels in das Myokard schießt, bestehend aus einem Generator (2) von unter Druck stehender Flüssigkeit, einem Vorrat (3) Arbeitsflüssigkeit und einem Handstück (4), der Generator (2) ist mit dem Flüssigkeitsvorrat und dem Handstück verbunden, **dadurch gekennzeichnet, dass** das Handstück (4) ein aktives Ende (7) aufweist, bestehend aus einem Schlauch (8) und einer Austrittsöffnung (9) der Arbeitsflüssigkeit, verbunden mit dem Generator (2) über einen Sequenzer (11) und einem Sauganschluss, der über ein Ansaugsystem (12) mit einer Unterdruckquelle (13) verbunden ist, **dadurch gekennzeichnet, dass** es einen Beschuss in Form einer oder mehrerer Impulse der unter Hochdruck stehenden Arbeitsflüssigkeit erzeugt und **dadurch**, dass die Arbeitsflüssigkeit ein Therapeutikum enthält.

2. Gerät nach Anspruch 1, **gekennzeichnet dadurch, dass** die Arbeitsflüssigkeit physiologische Kochsalzlösung enthält.

3. Gerät nach Anspruch 1, **dadurch gekennzeichnet, dass** die Arbeitsflüssigkeit eine Salzlösung oder eine Glukoselösung oder eine Ringer-Lactat-Lösung oder eine Hydroxy-Äthyl-Stärkelösung (HAES) oder eine Mischung dieser Lösungen enthält.

4. Gerät nach Anspruch 1, **dadurch gekennzeichnet, dass** das Therapeutikum ein Wirkstoff ist, der den Herzmuskel auf die Einbringung der Revaskularisationskanäle vorbereitet.

5. Gerät nach Anspruch 1, **dadurch gekennzeichnet, dass** das Therapeutikum ein Wirkstoff ist, der die Bildung neuer Gefäße fördert.

6. Gerät nach Anspruch 1, **dadurch gekennzeichnet, dass** das Therapeutikum ein vaskulärendothelialer Wachstumsfaktor ist.

7. Gerät nach Anspruch 1, **dadurch gekennzeichnet, dass** das Therapeutikum ein Wirkstoff für die Gentherapie-Behandlung ist.

8. Gerät nach Anspruch 1, **dadurch gekennzeichnet, dass** es über eine Möglichkeit zur Synchronisation der Impulse mit dem Herzrhythmus verfügt.

9. Gerät nach Anspruch 1, **dadurch gekennzeichnet, dass** der Vorrat (3) der Flüssigkeit sich in einem Weichbeutel befindet, der in einem unter Druck stehenden Behälter eingeschlossen ist.

10. Gerät nach Anspruch 1, **dadurch gekennzeichnet, dass** das aktive Ende (7) eine Vorrichtung zur Verankerung im Herzmuskel besitzt, zu dessen Immobilisation an der Stelle, wo ein Kanal zur Revaskularisation geschaffen werden soll.

11. Gerät nach Anspruch 1, **dadurch gekennzeichnet, dass** das aktive Ende (7) von einer Schürze, verbunden mit dem Ansaugsystem (12), umgeben ist, für sein Halten durch Unterdruck an der Herzwand, an der Stelle, wo ein Kanal zur Revaskularisation geschaffen werden soll.

12. Chirurgisches Gerät nach Anspruch 1,
**dadurch gekennzeichnet, dass** das aktive Ende (7) das eines biegsamen Schlauchs (38) ist, der in einem Führungskatheter (36) bis in das Herzinnere gleitet, wo ein Kanal zur Revaskularisation geschaffen werden soll.

13. Chirurgisches Gerät nach Anspruch 1, **dadurch gekennzeichnet, dass** das aktive Ende (7) geeignet ist in einen Trokar eingeführt zu werden, der durch den Thorax bis zum Bereich für den Eingriff führt.

14. Chirurgisches Gerät nach Anspruch 1, **dadurch gekennzeichnet, dass** der Kanal (8) für den Durchfluss der Arbeitsflüssigkeit am Ende einer Impulsion sofort mit dem Ansaugsystem (12) verbunden werden kann.

15. Chirurgisches Hochdruck-Flüssigkeitsstrahlgerät nach Anspruch 1, **dadurch gekennzeichnet, dass** der Druck der Arbeitsflüssigkeit zwischen 10 und 15 Bar beträgt.

16. Chirurgisches Gerät nach Anspruch 1, **dadurch gekennzeichnet, dass** der Durchmesser der Austrittsöffnung (9) der Arbeitsflüssigkeit zwischen 0,1 und 0,5 mm beträgt.

17. Chirurgisches Gerät nach Anspruch 1, **dadurch gekennzeichnet, dass** die Impulsdauer eines Beschusses mit nur einem Impuls der Arbeitsflüssigkeit zwischen 100 und 1000 ms beträgt.
